Europäisches Patentamt

European Patent Office

Office européen des brevets

(11) Numéro de publication: **0 633 768 B1**

# (12) FASCICULE DE BREVET EUROPEEN

(45) Date de publication du fascicule du brevet: **15.11.95**  (51) Int. Cl.⁶: **A61K 9/127**

(21) Numéro de dépôt: **93908988.4**

(22) Date de dépôt: **02.04.93**

(86) Numéro de dépôt internationale :
**PCT/FR93/00335**

(87) Numéro de publication internationale :
**WO 93/19735 (14.10.93 93/25)**

(54) **PROCEDE DE PREPARATION DE MICRO-CAPSULES OU DE LIPOSOMES DE TAILLES CONTROLEES.**

(30) Priorité: **03.04.92 FR 9204108**

(43) Date de publication de la demande:
**18.01.95 Bulletin 95/03**

(45) Mention de la délivrance du brevet:
**15.11.95 Bulletin 95/46**

(84) Etats contractants désignés:
**BE CH DE ES FR GB IT LI LU NL SE**

(56) Documents cités:
**EP-A- 0 466 236**
**WO-A-86/02264**
**DE-C- 4 038 075**
**GB-A- 1 376 166**

**H. SUCKER ET AL. 'PHARMAZEUTISCHE
TECHNOLOGIE' 1991 , GEORG THIEME VER-
LAG , STUTTGART (DE)**

(73) Titulaire: **CENTRE NATIONAL DE LA RECHER-
CHE SCIENTIFIOUE**
**15, quai Anatole France**
**F-75007 Paris (FR)**

(72) Inventeur: **ROUX, Didier**
**54, avenue Gambetta**
**F-33700 Mérignac (FR)**
Inventeur: **DIAT, Olivier**
**126, rue Belleville**
**F-33000 Bordeaux (FR)**
Inventeur: **LAVERSANNE, René**
**62, avenue du Parc-d'Espagne**
**F-33600 Pessac (FR)**

(74) Mandataire: **Le Guen, Gérard et al**
**CABINET LAVOIX**
**2, place d'Estienne d'Orves**
**F-75441 Paris Cédex 09 (FR)**

Rank Xerox (UK) Business Services
(3.10/3.09/3.3.3)

## Description

La présente invention a pour objet un procédé de préparation de micro-capsules ou de liposomes de taille contrôlée par application d'un cisaillement constant sur une phase lamellaire.

On entend par microcapsule une particule de taille micronique (0,1 à 10 $\mu$m), fermée par une ou plusieurs doubles couches (constituant la membrane) composées d'au moins un type de tensioactif (molécule composée d'une partie lipophile et une autre hydrophile se fixant aux interfaces). Cette ou ces membrane(s) enferme(nt) dans l'espace un volume de solvant isolé du reste de la solution qui est le volume encapsulé. Le rendement d'encapsulation est défini comme le pourcentage de volume encapsulé par rapport au volume total de solvant.

Dans le cas particulier de tensioactifs d'origines lipidiques, notamment des phospholipides, ces capsules sont appelées liposomes.

De nombreuses méthodes de préparation de micro-capsules et de liposomes ont été décrites dans la littérature. Parmi les méthodes proposées, on trouve notamment des méthodes basées sur la dispersion mécanique du ou des tensioactifs dans un solvant, des méthodes de préparation d'émulsions d'un solvant organique volatil dans un solvant aqueux puis évaporation de la partie organique, et des méthodes par polymérisation d'un monomère tel que l'acide acrylique (par exemple BE-A-808034, FR-A-2504408, US-A-3242051, US-A-4637905).

Dans le cas des liposomes, les procédés décrits comportent généralement des méthodes par émulsification (par exemple FR-A-2315991, FR-A-2399242 et FR-A-2521565).

Ces méthodes conduisent, dans les meilleurs cas, à des taux d'encapsulation de l'ordre de 50 % et à des particules relativement polydispersées.

Selon la présente invention, on propose une méthode simple de préparation de micro-capsules monodispersées très concentrées, à très haut rendement d'encapsulation (plus de 90 %) tout en contrôlant la taille des micro-capsules très précisément.

Ce résultat est obtenu en soumettant une phase lamellaire, cristal liquide, monophasique à un cisaillement constant homogène dans l'espace.

Ce résultat est étonnant car l'homme de l'art aurait logiquement pensé que l'application d'un cisaillement constant à une phase lamellaire aurait conduit à une orientation au moins partielle de cette phase plutôt qu'à la fabrication de petites particules isotropes de taille donnée .

L'invention a donc pour objet un procédé de préparation de micro-capsules de tailles contrôlées, dans lequel on prépare une phase lamellaire homogène cristal liquide comprenant au moins un tensioactif et au moins un solvant et, le cas échéant, une substance destinée à être encapsulée, caractérisé en ce que l'on soumet cette phase lamellaire à un cisaillement constant.

Dans une première étape on prépare une phase lamellaire homogène constituée d'au moins un type de tensioactif (ionique ou non ionique) dans au moins un type de solvant (notamment l'eau ou une solution aqueuse saline ou alcoolique). Une phase lamellaire est définie par un empilement périodique de membranes séparées par un solvant. C'est une phase cristal-liquide (smectique A) caractérisée par un ordre solide dans la direction perpendiculaire aux membranes et un ordre liquide dans les deux autres directions. Les concentrations sont choisies en fonction du diagramme de phase du système qui localise la zone de stabilité de la phase lamellaire. Généralement, cette phase lamellaire existe dans tous les systèmes tensioactifs/eau à des concentrations élevées de tensioactifs (> 30 % en poids). Dans certains cas cette phase lamellaire persiste pour des concentrations beaucoup plus faibles de tensioactifs (jusqu'à moins de 1-10 %).

En pratique on pourra utiliser notamment de 0,5 à 50 % en poids de tensioactifs par rapport à la phase lamellaire. Ces tensioactifs peuvent être aussi bien ioniques (dérivés d'acides gras éventuellement alcoxylés, sulfonates, dérivés à ammonium quaternaire, etc) que non ioniques (polyethers, polyalcools, etc) et plus généralement tout composé pouvant former une phase lamellaire et seront choisis en fonction des applications du produit préparé.

De plus, on peut préparer des systèmes où la membrane est constituée d'un film mince d'eau entouré par deux couches de tensioactifs (membranes inverses),le tout étant dilué dans un solvant hydrophobe.

Dans le cas où la membrane est constituée d'eau (membrane inverse), le solvant est choisi parmi les liquides hydrophobes, notamment les hydrocarbures aliphatiques (en particulier en $C_5$ à $C_{25}$) ou benzéniques, éventuellement halogénés, les alcools supérieurs (en particulier $C_4$ à $C_{12}$), les cétones, etc.

Cette phase lamellaire, une fois préparée, est aisément caractérisable par observation en microscopie optique polarisée de la texture montrant ainsi des défauts caractéristiques de l'ordre lamellaire (coniques focales, stries huileuses). Dans le cas des phases concentrées (> 20 %) on peut aussi la caractériser par rayons X. Dans le cas des phases diluées, on peut caractériser l'ordre lamellaire par diffusion des neutrons ou, dans les cas extrêmes, par diffusion de la lumière.

Dans une seconde étape, qui constitue l'originalité principale de l'invention, cette phase lamellaire est soumise à un cisaillement constant, dans un dispositif approprié. Il existe actuellement principalement deux types de dispositifs pouvant convenir à cet effet.

Un premier type de dispositif est une cellule dite de Couette, constituée de deux cylindres concentriques en rotation constante l'un par rapport à l'autre, où le cisaillement est défini par le rapport de la vitesse de déplacement relative divisée par la distance entre les cylindres. Un autre type de dispositif est la cellule de type plan/cône, où un cône dont la pointe est dirigée vers un plan, et dont l'axe est perpendiculaire à ce plan, tourne à une vitesse angulaire constante à distance du plan.

Dans les deux dispositifs décrits précédemment, on peut montrer que le cisaillement est constant dans l'ensemble de la cellule. Ces cellules sont couramment utilisées dans des appareils commerciaux, en particulier des rhéomètres permettant de mesurer les propriétés viscoélastiques de liquides (par exemple : CARRIMED ou RHEOMETRIX). Toutefois leur application à la préparation de micro-capsules n'a jamais été envisagée. De tels appareils peuvent être utilisés pour la préparation de micro-capsules selon l'invention.

La phase lamellaire doit être soumise à un cisaillement constant pendant un certain temps pour obtenir un état stationnaire. On peut suivre la cinétique de formation en mesurant en fonction du temps le couple qui s'applique sur l'un des cylindres lorsque l'on impose la vitesse de rotation de l'autre (i.e. le cisaillement). Ceci est facilement réalisé sur les appareils commerciaux décrits précédemment. Le temps typique pour atteindre l'état stationnaire est de l'ordre de quelques minutes à quelques heures, (notamment 1 mn à 100 min): plus le cisaillement est élevé plus le temps nécessaire est court. Le cisaillement est typiquement situé entre 1 et 1000 s$^{-1}$, notamment entre 2 et 400 s$^{-1}$.

Une fois le cisaillement arrêté, on récupère une crème constituée par un ensemble dense de sphérules monodisperses (objets sphériques) de phases lamellaires. Ces sphérules constituent les microcapsules dont la taille est une fonction directe du cisaillement qui a été imposé lors de la préparation. On peut montrer expérimentalement et théoriquement que le diamètre varie comme l'inverse de la racine carrée du cisaillement.

On peut mesurer la taille par différentes méthodes. La plus simple est de prélever un peu de crème et de remplir une cellule optiquement transparente (cellule Helma de 1 à 10 mm par exemple). En envoyant un faisceau laser à travers la cellule et en plaçant un écran sur le trajet après la cellule on remarque un anneau de diffusion dont la position donne directement le diamètre D des micro-capsules en utilisant la formule classique:

$$D = \lambda/n/2/\mathrm{Sin}(\theta/2)$$

$\theta$      étant l'angle formé par la position de l'anneau et le faisceau initial,

$\lambda$      étant la longueur d'onde de la lumière, et

$n$      étant l'indice de réfraction du milieu

On peut aussi placer la crème obtenue sous un microscope polarisant et observer une texture homogène dont la taille caractéristique est le diamètre des microcapsules.

On peut encore faire des images de microscopie électronique dans les mêmes conditions que celles que l'on utilise pour caractériser les liposomes.

Le procédé selon l'invention permet de préparer des micro-capsules ayant des dimensions généralement entre 0,1 et 50 micromètres, plus habituellement entre 0,8 et 8 micromètres, avec moins de 10 % de polydispersité en rayon. Il convient particulièrement à la préparation de liposomes.

A l'état non dilué, ces microcapsules sont très stables et peuvent être conservées très longtemps selon le tensioactif utilisé.

Les microcapsules préparées sous forme de crème par le procédé selon l'invention peuvent être par la suite utilisées directement pour préparer une solution diluée de micro capsules par simple addition de solvant. La stabilité des micro capsules en suspension est alors identique à celle obtenue par d'autres méthodes et est donc une fonction du système utilisé.

On peut mesurer le rendement d'encapsulation soit directement sur la crème par une méthode de conductivité à basse fréquence par exemple, soit par une technique classique sur une solution diluée de microcapsules. On peut également mesurer le rendement d'encapsulation en incorporant un colorant dans la phase lamellaire et en mesurant après cisaillement et centrifugation la concentration en colorant dans le surnageant. On obtient généralement une encapsulation de l'ordre de 90 à 95 %.

Le procédé de préparation selon l'invention permet donc l'obtention de micro capsules de tailles contrôlées et monodisperses. De plus, on obtient une concentration très élevée de ces particules. L'ensemble de ces propriétés permet une détermination facile de la taille caractéristique par l'observation d'un anneau de diffusion de la lumière ou même par mesure directe sous microscope à contraste de phase.

On peut expliquer la formation de ces sphérules de la façon suivante. Lorsque le cisaillement est très petit (typiquement < 1 s$^{-1}$) on obtient le régime d'orientation de la phase lamellaire décrit par Oswald et Kléman (J. de physique lettres 43, L-411, 1983) dans le cas des phases smectiques thermotropes. Le mouvement se fait alors par déplacement de défauts suivant les lois de la lubrification des smectiques. Dès que l'on dépasse un cisaillement critique (de l'ordre de 1 s$^{-1}$), le mouvement imposé est trop rapide pour permettre aux dislocations de bouger et le système forme des objets sphériques de tailles constantes qui roulent les uns sur les autres. La taille est fixée par un équilibre entre la force élastique nécessaire pour maintenir le système à une taille D et la force visqueuse qui s'exerce sur chacune des particules par ses voisines en déplacement. On peut montrer alors que :

$$D = \sqrt{\frac{4\pi(2k_c + \bar{k})}{\eta \, \dot{d} \, \gamma}}$$

avec $k_c$ et $\bar{k}$ qui sont respectivement les constantes élastiques de courbures moyenne et gaussienne de la membrane.

$\eta$ est la viscosité du milieu
$\dot{\gamma}$ est le cisaillement
d est la distance entre membranes dans la phase lamellaire d'origine.

Indépendamment des propriétés d'encapsulation décrites ci-dessus, la crème obtenue est un milieu viscoélastique à seuil.

La relation qui existe entre la taille des microcapsules obtenues et le cisaillement appliqué montre qu'il est possible de régler la taille en fonction des applications. Cela permet également de modifier les propriétés viscoélastiques du système sans changer sa composition simplement en modifiant la valeur du cisaillement. Il est ainsi possible de préparer des fluides viscoélastiques ayant différentes fréquences viscoélastiques, la fréquence viscoélastique étant définie comme la fréquence à laquelle les modules élastiques et visqueux se croisent.

Dans un mode de réalisation avantageux de l'invention, on incorpore dans la phase lamellaire cristal liquide, avant le cisaillement, un monomère à l'état dissous dans l'un des constituants de cette phase lamellaire et l'on initie la polymérisation du monomère après l'étape de cisaillement.

Le monomère peut par exemple être soit dissous dans l'eau (par exemple de l'acrylamide ou un dérivé de l'acide acrylique), soit dissous dans l'huile (styrène par exemple), soit, s'il a des propriétés tensioactives, solubilisé dans la membrane de tensioactif. On peut aussi utiliser le monomère à l'état pur afin de remplacer l'un des constituants de la phase lamellaire. C'est le cas par exemple pour l'huile qui peut être du styrène pur, ou pour le tensioactif qui peut être un tensioactif polymérisable utilisé pur. Généralement, on ajoute un agent réticulant permettant d'obtenir un gel de polymère stable.

Selon la nature de la réaction d'initiation de la polymérisation, il peut être nécessaire d'ajouter un initiateur chimique. Il faut dans ce cas l'ajouter avant l'étape de cisaillement, afin d'assurer l'homogénéité de la solubilisation. Il est alors possible de déclencher la réaction de polymérisation par la modification d'un paramètre extérieur (par exemple par chauffage ou exposition à un rayonnement ultraviolet) en évitant tout démarrage de la réaction pendant la phase de préparation des microcapsules.

De la même manière, si l'on désire encapsuler un principe actif, il doit être dissous dans la phase lamellaire, avant son cisaillement.

Cette phase, contenant le principe actif, le monomère et l'initiateur, est soumise à un cisaillement par le procédé selon l'invention, constant pendant la durée nécessaire à l'obtention de l'état stationnaire. Ceci constitue la première étape. A l'issue de ce traitement on récupère la crème obtenue.

Dans une seconde étape, cette crème est polymérisée. La polymérisation peut être effectuée soit sur la crème pure, soit diluée dans le solvant ayant été utilisé pour la fabrication de la phase cristal-liquide. Le déclenchement de la réaction de polymérisation permet d'obtenir des microcapsules polymérisées. On peut alors diluer ou utiliser ces microcapsules telles quelles.

Ces microcapsules polymérisées se caractérisent entre autres par une stabilité beaucoup plus grande que celle des capsules non polymérisées (pas de dégradation après plusieurs mois) et un ralentissement notable de la fuite du principe actif inclus dans les capsules.

On peut dans certains cas disperser ces microparticules aussi bien dans un solvant aqueux qu'organique.

Dans un autre mode de réalisation de l'invention on utilise comme phase lamellaire une phase qui est susceptible de passer de l'état de phase lamellaire cristal-liquide (phase L$\alpha$) à une phase gel (phase L$\beta$) à plus basse température où les molécules de tensioactifs sont organisées selon un ordre solide bidimensionnel dans chaque membrane et après cisaillement on porte les microcapsules à une température inférieure à la température de transition de phase gel/liquide.

Cette transition de phase est bien connue dans les systèmes lipidiques mais aussi pour les tensioactifs synthétiques.

Les microcapsules sont préparées en phase lamellaire cristal-liquide (phase haute température) en suivant le procédé décrit précédemment. Les principes de formation et les résultats sont similaires à ceux décrits auparavant. La crème de microcapsules concentrées est ensuite portée à une température inférieure à celle de la transition gel/liquide. On obtient ainsi une crème concentrée de microcapsules solidifées en phase gel. On peut alors diluer ces microcapsules dans un solvant. Si un principe actif est ajouté lors de la préparation de la phase cristal-liquide de départ, on retrouve ce principe actif dans les capsules solides à l'issue de la préparation En réchauffant la suspension diluée de capsules solides au-dessus de la température de transition gel/liquide, ce principe est alors libéré selon une cinétique liée à la composition de la membrane en phase cristal-liquide.

On peut également utiliser la propriété de certains gels physiques de passer de l'état liquide à l'état gel en fonction de la température et de façon réversible pour permettre la fabrication de microparticules gélifiées. On peut ainsi préparer une phase cristal-liquide avec dans le solvant le polymère gélifiant. Cette phase est cisaillée au-dessus de la température de gélification du polymère puis, après obtention des microcapsules, refroidi en-dessous. Les capsules obtenues peuvent être alors dispersées dans un solvant. En reproduisant la transition gel/liquide en sens inverse on peut ainsi contrôler le relargage d'un principe actif (encapsulé lors de la préparation).

On peut en outre coupler le procédé selon l'invention à un procédé classique d'encapsulation : la coacervation. Usuellement la coacervation consiste dans une première étape à préparer une émulsion d'un liquide hydrophobe dans de l'eau. Puis un polymère est adsorbé à l'interface huile/eau pour former une coquille polymérisée permettant de stabiliser cette émulsion. On encapsule donc par ce procédé un principe actif hydrophobe dans une capsule hydrophile. De plus la taille des microcapsules ainsi obtenue est relativement grande (10-1000 $\mu$m).

Si l'on applique la même méthode dans une seconde étape au procédé selon l'invention, on peut encapsuler un composé hydrophile dans une matrice hydrophile (ou un composé hydrophobe dans une capsule hydrophobe). De plus, la taille est bien contrôlée et peut descendre en-dessous du micromètre.

On peut en outre utiliser le procédé selon l'invention pour préparer des particules solides. A cet effet, on utilise le procédé selon l'invention comme étape préparatoire d'un micro-réacteur chimique afin de préparer par exemple des particules solides de taille contrôlée. On illustrera le procédé par la préparation de particules de nickel monodispersées. Deux méthodologies sont appliquées. Si une réaction chimique consiste à faire réagir une molécule A sur une molécule B (ou un ensemble de molécules) on peut encapsuler l'un des réactifs dans les microcapsules et disperser ces capsules dans un solvant contenant le réactif B. La réaction est alors déclenchée au niveau de la capsule servant de réservoir (micro-réacteur). Si cette réaction consiste en la production d'un produit AB solide ou polymérisé, la taille de l'objet résultant est alors contrôlée par la quantité de produit réactif dans la capsule, par le nombre de sites réactifs et par la concentration du réactif à l'extérieur de la capsule. On peut aussi dans le cas d'une réaction catalytique, encapsuler le catalyseur dans la capsule et mettre l'ensemble des réactifs (sauf le catalyseur) dans le solvant servant de diluant. La dispersion des capsules contenant le catalyseur dans le solvant contenant les réactifs entraîne le déclenchement de la réaction au niveau de chaque capsule. De nouveau si on prépare des capsules solides ou polymérisées on peut ainsi contrôler la taille des particules résultantes.

Le procédé de préparation de microcapsules selon l'invention trouve des applications dans de nombreux domaines.

1) Peintures

Le procédé peut être utilisé à plusieurs niveaux dans la préparation de peintures. On peut encapsuler un principe actif et diluer ces capsules dans une peinture. Le relargage de ce principe actif est contrôlé par la taille des capsules, la polymérisation éventuelle et aussi des procédés spécifiques tels que ceux décrits plus haut (transition de phase, gels, coacervation, etc...). On peut aussi incorporer des particules solides préparées comme décrit ci-dessus afin de donner aux peintures des propriétés optiques ou diélectriques. Le principe actif peut être un insecticide, un fongicide ou tout autre produit demandant un relargage contrôlé dans le temps. On peut aussi mélanger des capsules de natures différentes afin de mieux répartir dans le temps la fuite du principe actif.

2) Produits phytosanitaires

Il est possible d'appliquer le procédé selon l'invention dans le domaine phytosanitaire, afin de traiter des produits végétaux. Deux actions peuvent être envisagées : d'une part, comme décrit précédemment, le relargage d'un principe actif peut être contrôlé. De plus, du fait de la nature des parois des capsules (tensioactives) et selon leur taille, ces capsules peuvent passer plus facilement la barrière protectrice des plantes. On peut ainsi encapsuler des pesticides ou des vitamines.

3) Reprographie

Dans le domaine de la reprographie, ce procédé permet l'encapsulation de colorants dans la préparation de films couleur. On peut aussi penser utiliser la possibilité de contrôler la taille de préparation des particules solides afin de préparer des grains de sel d'argent de taille contrôlée.

### 4) Cosmétique

Dans le domaine des cosmétiques, le procédé est directement applicable à la préparation de capsules enfermant un principe actif. On peut utiliser comme tensioactif des lipides, des acides gras, des tensioactifs non ioniques ou des dérivés de sucres. La possibilité de relargage contrôlé par le contrôle de la température (transition gel/liquide) peut se révéler particulièrement intéressante en utilisant une membrane qui a une température de transition au-dessous de 30/35° C. Les propriétés viscoélastiques peuvent aider à la formulation de crèmes de beauté.

### 5) Lessives liquides

Dans le domaine des lessives liquides, on peut penser utiliser le procédé selon l'invention afin de contrôler des réactions chimiques devant se dérouler lors du lavage. On peut aussi faire entrer ces microcapsules dans la constitution de produits adoucissants (en particulier ceux donnant des capsules en phase solide $L\beta$).

### 6) Produits agro-alimentaires

Dans le domaine agro-alimentaire, le procédé est utilisable comme alternative aux procédés à base de polymère. On remarquera que le très fort taux d'encapsulation ainsi que le contrôle précis de la taille sont des atouts importants. Les principes actifs à encapsuler sont des arômes ou tout autre produit nécessitant une protection particulière (produits sucrants par exemple).

### 7) Biomédical/pharmacie

Dans le domaine du biomédical et de la pharmacie, le procédé peut être appliqué dans de nombreuses voies.

L'encapsulation de principes actifs médicamenteux ou substances biologiques peut être obtenue en dissolvant dans la phase lamellaire de départ un ou plusieurs principes actifs. Ceux-ci se retrouvent ainsi encapsulés à l'intérieur des micro-capsules dans la proportion du rendement d'encapsulation.

Par ailleurs, on peut citer comme exemples la vectorisation de médicaments, la mise au point de produits de contraste pour l'imagerie médicale (produits magnétiques pour l'imagerie à résonance magnétique), préparation de sang artificiel (en utilisant des tensioactifs fluorés). On peut aussi utiliser ce procédé dans la préparation de tests médicaux (en utilisant par exemple la polymérisation).

### 8) Liants hydrauliques

Le procédé permet par exemple de préparer un catalyseur retard pour la prise rapide de matériaux tels que ciments, béton, plâtre ... En encapsulant ce catalyseur par le procédé selon l'invention on peut retarder son effet. Ceci permet la mise en oeuvre du matériau et le déclenchement retardé de la prise dû à la fuite contrôlée du catalyseur.

Les exemples suivants illustrent l'invention avec les figures annexées sur lesquelles :
- la fig. 1 est une photographie au microscope a contraste de phase de micro-capsules obtenues selon le procédé de l'invention,
- la fig. 2 représente la mesure de la taille des microcapsules en fonction du cisaillement appliqué selon le procédé de l'invention, et
- la fig. 3 est un diagramme correspondant à la région de formation des micro-capsules en fonction de la proportion de solvant et du taux de cisaillement.

Exemple 1 : Tensioactif ionique + eau salée

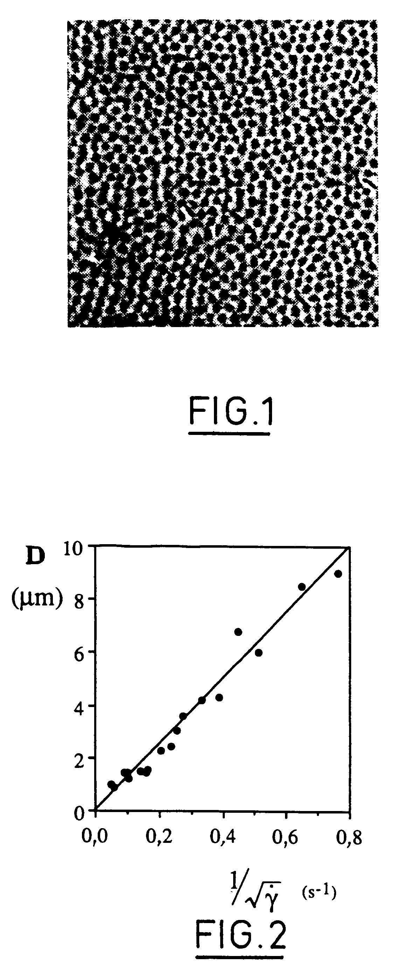

On prépare une phase lamellaire par dissolution de 16,8 % d'un tensioactif ionique, le di-octyl sulfosuccinate de sodium, (Aérosol OT de la société SIGMA Chemical Co) dans 83,2 % d'eau salée (chlorure de sodium à 12 g/l). Cette phase lamellaire est ensuite soumise à un cisaillement constant de 3 $s^{-1}$ pendant 30 min, à l'aide d'un rhéomètre (CARRIMED 50) équipé d'une cellule de Couette de Mooney. La crème obtenue est transvasée dans une cellule transparente de 1 mm placée dans un faisceau laser. La taille de l'anneau de diffusion observée indique que les liposomes obtenus ont un diamètre de 2 $\mu$m avec une polydispersité d'environ 10 %. On peut observer sous microscope polarisant une texture homogène de taille caractéristique de 2 $\mu$m . On peut diluer cette crème dans de l'eau salée à 12 g/l et observer sous microscope à contraste de phase une solution plus ou moins concentrée de micro-capsules (figure 1)

Exemple 2 : Variation de la taille des microcapsules en fonction du cisaillement.

On prépare une phase lamellaire par dissolution de 17 % de tensioactif ionique (Aérosol OT de la société SIGMA) dans 83 % d'eau salée (chlorure de sodium) à 15 g/l. Cette phase lamellaire est ensuite soumise à un cisaillement variant de 2 à 400 $s^{-1}$ comme dans l'exemple 1. La taille des micro-capsules est mesurée par diffusion de la lumière; on obtient la courbe présentée sur la figure 2 en annexe. La taille (diamètre) varie linéaire-

ment en fonction de l'inverse de la racine carrée du cisaillement de 8μm à 0,8μm.

Exemple 3 : Membrane inverse

On prépare une phase lamellaire inverse par mélange de 14,85 % de pentanol, 13,77 % de SDS (dodecylsulfate de sodium), 50,06 % de dodecane et 21,32 % d'eau et homogénéisation. Après l'avoir laissé reposer, cette phase lamellaire est composée de films d'eau entourés de tensioactifs d'épaisseur 20 Å et séparés par un solvant composé de dodécane et de pentanol d'épaisseur 90 Å (caractérisation obtenue par la mesure du pic de Bragg en diffraction des rayons X). Cette phase est soumise à un cisaillement constant (entre 3 s$^{-1}$ et 280 s$^{-1}$). Pour chaque cisaillement, on mesure à l'état stationnaire la taille par diffusion de la lumière. On obtient une taille variant de D = 1 μm à 6 μm en fonction du cisaillement.

Exemple 4 : Tensioactif non ionique + alcool + eau pure.

Une phase lamellaire composée de 16 % (en poids)de tensioactif C 12 E5 (mono n-dodécyl-éther de pentaéthylèneglycol de la société NIKKOL), de 4,25 % d'hexanol et de 79,75 % d'eau est préparée puis soumise à un cisaillement pendant 10 min à 2 s$^{-1}$. On obtient une crème composée de sphérules ayant 2μm de diamètre qui peut être mesurée par diffusion de la lumière par la méthode décrite dans l'exemple 1. La variation du cisaillement de 1s$^{-1}$ à 10s$^{-1}$ permet d'obtenir des tailles variant de 1,5 à 8μm.

Exemple 5 : Tensioactif ionique + eau pure

Une phase très diluée de DDAB (bromure de didodécyl-diméthyl-ammonium de la société ALDRICH) dans l'eau pure est préparée (5 % de DDAB dans 95 % d'eau), ce qui correspond à une distance entre membranes de 800 Å (mesurée par diffusion des neutrons). Cette phase soumise à un cisaillement constant de 10 s$^{-1}$ pendant 30 min conduit à une phase concentrée de sphérules d'environ 1μm de diamètre.

Exemple 6 : Lécithine + cholesterol + eau

Un mélange de 47 % en poids de lécithine de soja (Cernes Synthelabo), 13 % de cholestérol et de 40 % d'eau est préparé puis soumis à un cisaillement constant pendant 10 min à 400 s$^{-1}$. On obtient une phase de micro-capsules concentrées de 2μm de diamètre. On peut disperser ces sphérules dans de l'eau pure et observer le mouvement brownien de ces liposomes ainsi que leur taille avec un microscope à contraste de phase. En faisant varier le cisaillement de 300 à 700 s$^{-1}$, on peut faire varier la taille des liposomes de 3 à 1μm.

Exemple 7 : Diagramme de formation des micro-capsules

Afin de déterminer la possibilité de formation de micro-capsules pour un système donné, on peut établir un diagramme d'orientation qui délimite la zone d'existence de ces micro-capsules en fonction du cisaillement et d'autres paramètres sur lesquels on peut jouer expérimentalement. A titre d'exemple on a étudié systématiquement un système identique à l'exemple 3. La région de formation de micro-capsules a été localisée en fonction de deux paramètres : le cisaillement et le taux de dilution de la phase lamellaire qui détermine la distance entre membranes. La phase lamellaire inverse de départ contient : 22 % de pentanol, 31 % de SDS et 47 % d'eau. Cette phase est diluée avec un mélange de 91 % de dodecane et 9 % de pentanol. La phase lamellaire est stable de 0 % de dodecane à 80 % de dodecane.

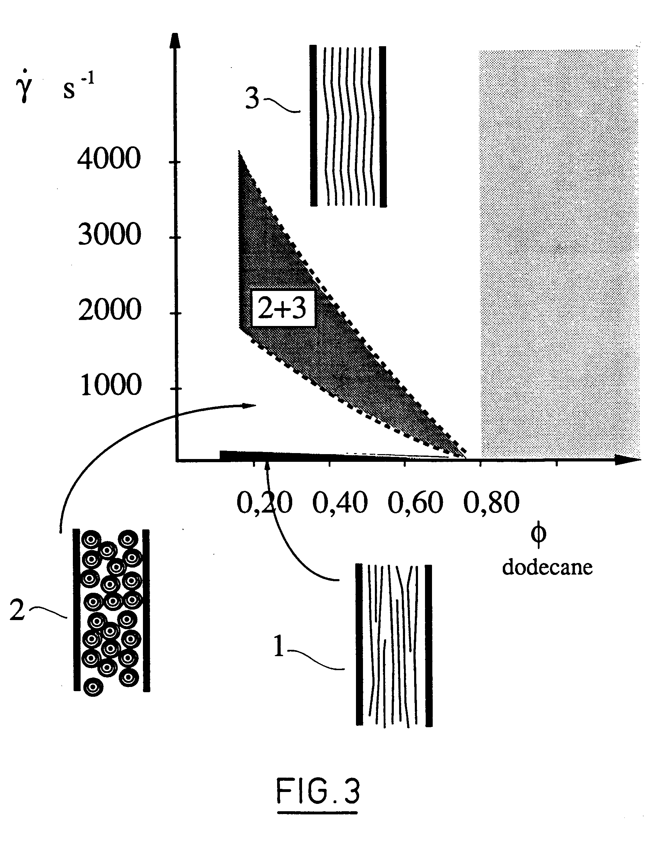

La figure 3 en annexe représente la région de formation des micro-capsules en fonction de la fraction volumique de dodécane et du cisaillement. Ce diagramme a été obtenu avec une cellule de Couette de 2 mm. Sur cette figure la région 2 où les micro-capsules se forment, est limitée par deux lignes qui correspondent respectivement à une région globalement orientée avec les membranes parallèles à la direction de l'écoulement avec défauts (région 1 à bas cisaillement) ou sans défaut (région 3 à haut cisaillement) dans cette direction.

EXEMPLE 8

Préparation de microcapsules polymérisées.

On prépare une phase lamellaire contenant en poids : 30 % d'Aérosol OT, 60 % d'eau salée (15 g/l de NaCl), 9 % d'acrylamide et 1 % de méthylène bis-acrylamide (agent réticulant). On ajoute à 1 g de cette préparation 50 μl d'une solution de triéthanolamine (60 gl$^{-1}$) dans l'eau et 50 μl d'une solution contenant 0,2 gl$^{-1}$ de bleu de méthylène et 0,2 gl$^{-1}$ d'éosine (initiateur de la réaction de polymérisation en présence de lumière). On a soin de ne pas exposer le mélange à la lumière pendant l'étape de préparation des microcapsules. Cette phase cristal-liquide est placée dans une cellule de Couette (ou plan-cône) et soumise à un cisaillement constant de 20 s$^{-1}$ pendant 2 heures. Après cette étape, la crème ainsi obtenue est placée dans une cellule en quartz et soumise à un rayonnement lumineux (lumière solaire ou lampe à vapeur de mercure) pendant quelques minutes. Il se produit alors une décoloration progressive indiquant la consommation des initiateurs et le démarrage de la réaction. On récupère alors une crème de microcapsules polymérisées. Ces microcapsules peuvent être diluées dans une solution d'eau salée (15 g/l) et observées au microscope optique. On peut aussi diluer ces microcapsules dans du cyclohexane. On obtient alors des petites capsules de polymère en suspension dans une phase inverse. Une mesure par diffusion dynamique de la lumière indique que ces particules ont un diamètre de 0,2 µm.

En variante, on peut diluer la crème d'un facteur deux (dans l'eau salée à 15 g/l de NaCl) avant de procéder à l'étape de polymérisation. On obtient alors des microcapsules similaires.

EXEMPLE 9

Préparation de microcapsules polymérisées

On prépare un mélange contenant 30 % d'Aérosol OT, 50 % d'eau salée (15 g/l de NaCl), 15 % d'acrylamide et 5 % de méthylène bis-acrylamide (agent réticulant). On ajoute à 1 g de cette préparation 50 µl d'une solution de triéthanolamine (60 gl$^{-1}$) dans l'eau et 50 µl d'une solution contenant 0,2 gl$^{-1}$ de bleu de méthylène et 0,2 gl$^{-1}$ d'éosine (initiateur de la réaction de polymérisation en présence de lumière). On soumet cette phase au cisaillement puis à l'action d'un rayonnement ultraviolet et on obtient des microparticules. Ces microcapsules sont plus stables que dans l'exemple 9 et leur taille reste constante avec le temps.

EXEMPLE 10

Préparation de microcapsules à transition de phase.

On prépare une phase contenant en poids 10 % de SDS (dodécyl sulfate de sodium), 10 % de dodécanol et 80 % d'eau salée à 20 g/l (on peut ajouter dans cette eau un principe actif, par exemple de la calcéine (agent fluorescent) pour la démonstration de l'effet indiqué). Cette phase est cisaillée à une température de 50°C pendant 15 min. sous un cisaillement de 20 s$^{-1}$. Puis on refroidit la crème prélevée à une température de 20°C. On peut alors diluer les capsules obtenues dans une phase d'eau salée (20 g/l) et l'on obtient une suspension de particules solides avec le principe actif encapsulé. En réchauffant cette suspension au-dessus du point de gel (40°C environ) celui-ci est libéré. Dans le cas où le principe actif est de la calcéine la libération peut être suivie par fluorescence avec la présence d'un agent inhibant la fluorescence dans l'eau de dilution (sel de cobalt par exemple).

EXEMPLE 11

Préparation de particules de nickel colloïdal de taille contrôlée.

A 1 g d'une phase lamellaire contenant 17 % en masse d'Aérosol OT et 83 % d'eau salée à 15 g/l, on ajoute 0,1 ml d'une solution 10$^{-2}$M de tétrachloropalladate de sodium. Cette phase est cisaillée à 4 s$^{-1}$ pendant 2 h. On disperse 0,2 g de la crème de sphérulites obtenue dans 2 ml d'eau salée à 15 g/l, puis on y ajoute 1 ml d'une solution à 5 % en masse de diméthylamine-borane. Après quelques minutes on ajoute 1 ml d'une solution contenant 0,1 mole/l de chlorure de nickel II, 0,1 mol/l de gluconate de sodium, 0,2 mol/l d'hypophosphite de sodium et 3,8 % en volume d'ammoniac concentré. La solution noircit immédiatement et un dégagement gazeux apparaît. Les grains de nickel peuvent être recueillis par centrifugation. L'étude par diffraction des rayons X indique une taille de 300 ± 25 Å.

**Revendications**

1. Procédé de préparation de microcapsules de tailles contrôlées, dans lequel on prépare une phase lamellaire homogène cristal liquide, monophasique, comprenant au moins un tensioactif et au moins un solvant et, le cas échéant, une substance destinée à être encapsulée et formant un empilement de membranes, caractérisé en ce que l'on soumet cette phase lamellaire à un cisaillement constant.

2. Procédé selon la revendication 1, caractérisé en ce que le solvant est l'eau ou une solution aqueuse saline.

3. Procédé selon la revendication 1 ou 2, caractérisé en ce que les microcapsules sont des liposomes.

4. Procédé selon la revendication 1, caractérisé en ce que les membranes sont des membranes inverses formées d'eau entourée de tensioactif dans un solvant hydrophobe.

5. Procédé selon l'une quelconque des revendications 1 à 4, caractérisé en ce que le(s) tensioactif(s) constitue(nt) de 0,5 à 50 % en poids de la phase lamellaire.

6. Procédé selon l'une quelconque des revendications 1 à 5, caractérisé en ce que le cisaillement est entre 1 et 1000 s$^{-1}$.

**7.** Procédé selon la revendication 6, caractérisé en ce que le cisaillement est entre 2 et 400 s$^{-1}$.

**8.** Procédé selon l'une des revendications 1 à 7, caractérisé en ce que le cisaillement constant est réalisé à l'aide d'une cellule constituée de deux cylindres concentriques en rotation constante l'un par rapport à l'autre.

**9.** Procédé selon l'une quelconque des revendications 1 à 7, caractérisé en ce que le cisaillement constant est réalisé à l'aide d'une cellule constituée par un plan/cône.

**10.** Procédé selon l'une quelconque des revendications 1 à 9, caractérisé en ce que l'on incorpore dans la phase lamellaire cristal liquide avant le cisaillement, un monomère à l'état dissous dans l'un des constituants de cette phase lamellaire et l'on initie la polymérisation du monomère après le cisaillement.

**11.** Procédé selon l'une quelconque des revendications 1 à 10, caractérisé en ce que l'on utilise une phase lamellaire susceptible de passer à l'état de phase gel à plus basse température et après cisaillement, on porte les microcapsules à une température inférieure à la température de transition de phase gel/liquide.

**12.** Procédé selon l'une quelconque des revendications 1 à 11, caractérisé en ce que les microcapsules ont un diamètre de 0,1 à 10 $\mu$m.

## Claims

**1.** Method for preparing microcapsules of controlled sizes, in which a single-phase liquid crystal homogeneous lamellar phase is prepared, comprising at least one surfactant and at least one solvent and, where appropriate, a substance intended to be encapsulated and forming a stack of membranes, characterised in that this lamellar phase is subjected to constant shearing.

**2.** Method according to Claim 1, characterised in that the solvent is water or a saline aqueous solution.

**3.** Method according to Claim 1 or 2, characterised in that the microcapsules are liposomes.

**4.** Method according to Claim 1, characterised in that the membranes are reverse membranes formed by water surrounded by surfactants in a hydrophobic solvent.

**5.** Method according to any one of Claims 1 to 4, characterised in that the surfactant or surfactants constitute from 0.5 to 50% by weight of the lamellar phase.

**6.** Method according to any one of Claims 1 to 5, characterised in that the shearing is between 1 and 1000 s$^{-1}$.

**7.** Method according to Claim 6, characterised in that the shearing is between 2 and 400 s$^{-1}$.

**8.** Method according to any one of Claims 1 to 7, characterised in that the constant shearing is produced by means of a unit consisting of two concentric cylinders rotating constantly with respect to each other.

**9.** Method according to any one of Claims 1 to 7, characterised in that the constant shearing is produced by means of a unit formed by a plane/cone.

**10.** Method according to any one of Claims 1 to 9, characterised in that, in the liquid crystal lamellar phase before shearing, there is incorporated a monomer in the dissolved state in one of the constituents of this lamellar phase, and the polymerisation of the monomer is initiated after the shearing.

**11.** Method according to any one of Claims 1 to 10, characterised in that use is made of a lamellar phase able to change to the gel phase state at a lower temperature, and after shearing the microcapsules are taken to a temperature lower than the gel/liquid phase transition temperature.

**12.** Method according to any one of Claims 1 to 11, characterised in that the microcapsules have a diameter of 0.1 to 10 $\mu$m.

## Patentansprüche

**1.** Verfahren zur Herstellung von Mikrokapseln mit kontrollierten(gesteuerten) Abmessungen-(Formen), bei dem man eine lamellenförmige, homogene, einphasige Flüssigkristall-Phase herstellt, die mindestens ein oberflächenaktives Agens und mindestens ein Lösungsmittel sowie gegebenenfalls eine Substanz, die eingekapselt werden soll, enthält und einen Stapel von Membranen bildet, dadurch gekennzeichnet, daß man diese lamellenförmige Phase einer konstanten Scherkraft aussetzt.

2.	Verfahren nach Anspruch 1, dadurch gekennzeichnet, daß das Lösungsmittel Wasser oder eine wäßrige Salzlösung ist.

3.	Verfahren nach Anspruch 1 oder 2, dadurch gekennzeichnet, daß die Mikrokapseln Liposome sind.

4.	Verfahren nach Anspruch 1, dadurch gekennzeichnet, daß die Membranen inverse Membranen sind, die gebildet werden von Wasser, umgeben von einem oberflächenaktiven Agens, in einem hydrophoben Lösungsmittel.

5.	Verfahren nach einem der Ansprüche 1 bis 4, dadurch gekennzeichnet, daß das(die) oberflächenaktive(n) Agens (Agentien) 0,5 bis 50 Gew.-% er lamellenförmigen Phase darstellen.

6.	Verfahren nach einem der Ansprüche 1 bis 5, dadurch gekennzeichnet, daß die Scherkraft zwischen 1 und 1000 $s^{-1}$ liegt.

7.	Verfahren nach Anspruch 6, dadurch gekennzeichnet, daß die Scherkraft zwischen 2 und 400 $s^{-1}$ liegt.

8.	Verfahren nach einem der Ansprüche 1 bis 7, dadurch gekennzeichnet, daß die konstante Scherkraft mit Hilfe einer Zelle erzeugt wird, die aus zwei konzentrischen Zylindern besteht, die sich mit konstanter Geschwindigkeit gegeneinander drehen.

9.	Verfahren nach einem der Ansprüche 1 bis 7, dadurch gekennzeichnet, daß die konstante Scherkraft mit Hilfe einer Zelle erzeugt wird, die besteht aus einer Ebene und einem Kegel.

10.	Verfahren nach einem der Ansprüche 1 bis 9, dadurch gekennzeichnet, daß man der lamellenförmigen Flüssigkristall-Phase vor dem Einwirkenlassen einer Scherkraft ein Monomer, gelöst in einem der Bestandteile dieser lamellenförmigen Phase, zusetzt und die Polymerisation des Monomers nach dem Einwirkenlassen der Scherkraft initiiert.

11.	Verfahren nach einem der Ansprüche 1 bis 10, dadurch gekennzeichnet, daß man eine lamellenförmige Phase verwendet, die bei tieferer Temperatur in eine Gelphase übergehen kann, und daß man nach dem Einwirkenlassen der Scherkraft die Mikrokapseln auf eine Temperatur unterhalb der Gel/Flüssigkeits-Phasenübergangstemperatur bringt.

12.	Verfahren nach einem der Ansprüche 1 bis 11, dadurch gekennzeichnet, daß die Mikrokapseln einen Durchmesser von 0,1 bis 10 µm haben.

## FIG.1

## FIG.2

FIG.3